# EUROPÄISCHE PATENTSCHRIFT

(11) **EP 2 686 294 B1**
(45) Veröffentlichungstag und Bekanntmachung des Hinweises auf die Patenterteilung: **16.08.2017**
(21) Anmeldenummer: 12708337.6
(22) Anmeldetag: 14.03.2012
(51) Int. Cl.: C07C 209/36, C07C 211/50, B01J 19/26

(54) **OPTIMIERTE DOSIERUNG DER EDUKTE FÜR EIN VERFAHREN ZUR HERSTELLUNG VON AROMATISCHEN AMINEN DURCH HYDRIERUNG VON NITROAROMATEN**
OPTIMIZED METERING OF REACTANTS FOR A PROCESS FOR PREPARING AROMATIC AMINES BY HYDROGENATION OF NITROAROMATICS
DOSAGE OPTIMISÉ DES ÉDUITS POUR UN PROCÉDÉ DE FABRICATION D'AMINES AROMATIQUES PAR HYDROGÉNATION DE NITROAROMATES

(30) Priorität: 16.03.2011 EP 11158462
(43) Veröffentlichungstag der Anmeldung: 22.01.2014
(73) Patentinhaber: BASF SE, 67056 Ludwigshafen am Rhein (DE)
(72) Erfinder: WATERS, Gerrit, 76133 Karlsruhe (DE); BÜTTNER, Johannes, 01945 Ruhland (DE); RAICHLE, Andreas, 67063 Ludwigshafen (DE); HILLER, Markus, 67133 Maxdorf (DE); RENZE, Peter, 68163 Mannheim (DE); HERRICH, Michael, 01945 Jannowitz (DE); RÜGER, Helmut, 01993 Schipkau (DE); HANTEL, Burkhard, 01936 Schwepnitz (DE); HOFMANN, Arndt, 01445 Radebeul (DE); FRITZ, Rüdiger, 02994 Bernsdorf (DE); RICHTER, Kathrin, 01279 Dresden (DE); BRAUNSBERG, Holgar, 01968 Senftenberg (DE); BRUNTSCH, Lothar, 04932 Grossthiemig (DE)
(86) Internationale Anmeldenummer: PCT/EP2012/054414
(87) Internationale Veröffentlichungsnummer: WO 2012/123469

(56) Entgegenhaltungen:
- EP-A1- 0 634 391
- WO-A1-03/068724
- DE-C1- 19 844 901

## Beschreibung

Die Erfindung betrifft ein Verfahren zur Herstellung von aromatischen Aminen durch Hydrierung von entsprechenden Nitroaromaten mit Wasserstoff, sowie die Verwendung einer dafür geeigneten Vorrichtung. Insbesondere betrifft die Erfindung ein Verfahren zur Herstellung von Toluylendiamin (TDA) durch Hydrierung von Dinitrotoluol (DNT).

Toluylendiamin ist ein in der Technik häufig eingesetztes aromatisches Amin. Es wird insbesondere zu Toluylendiisocyanat weiterverarbeitet, das überwiegend in der Polyurethanherstellung Verwendung findet. Toluylendiamin wird durch katalytische Hydrierung von Dinitrotoluol hergestellt.

Bei der Herstellung von Toluylendiamin aus entsprechenden Dinitrotoluolen ist es möglich, dass ab einer bestimmten Temperatur und Konzentration eine selbstbeschleunigende Zersetzung von Dinitrotoluol erfolgt, was bei technisch durchgeführten Verfahren ein Sicherheitsrisiko darstellt. Ein weiteres Problem bei der großtechnischen Herstellung von Toluylendiaminen aus Dinitrotoluolen ist, dass aufgrund der Stoffeigenschaften der beteiligten Substanzen sowie aufgrund der Gegenwart eines suspendierten Katalysators lokale Verblockungen von Reaktorelementen entstehen können. Ein weiteres Problem bei der großtechnischen Herstellung von Toluylendiaminen aus Dinitrotoluolen ist, dass erhöhte DNT-Konzentrationen in der Reaktionsmischung den Katalysator schädigen können. Zur Minimierung des Katalysatorverbrauchs ist eine möglichst schnelle Einmischung des frisch zugeführten DNT unter Vermeidung signifikanter lokaler Überkonzentrationen günstig.

Verfahren zur Herstellung entsprechender Toluylendiamine durch Hydrierung von Dinitrotoluolen sind aus dem Stand der Technik bereits bekannt.

WO 98/55216 A1 offenbart einen Rohbündelreaktor für rasche, stark exotherme Reaktionen. Insbesondere ist dieser Reaktor zur Sumpfphasenhydrierung von Dinitrotoluol zu den entsprechenden Diaminen geeignet. Das Problem einer zu hohen Konzentration an Dinitrotoluolen wird in diesem Dokument nicht beschrieben, da der Focus dieses Dokumentes darauf liegt, die bei der stark exothermen Reaktion entstehende Reaktionswärme möglichst effizient abführen zu können.

JP 2008/222623 offenbart ein Verfahren zum gesteuerten Abbrechen von organischen Reaktionen, insbesondere der Hydrierung entsprechender aromatischer Nitroverbinddungen zu den entsprechenden aromatischen Aminen. Dazu wird zunächst die Zugabe der entsprechenden Dinitroverbindung und des Katalysators unterbrochen. Erst dann wird die Zugabe von Wasserstoff unterbrochen. So wird es gemäß dem genannten Dokument erreicht, dass keine risikobehaftete hohe Konzentration an Dinitrotoluolen im Reaktor auftritt.

US 2001/0027257 A1 offenbart ein Verfahren zur kontinuierlichen Hydrierung aromatischer Nitroverbindungen in Gegenwart eines geeigneten Katalysators. Die Konzentration der aromatischen Nitroverbindung soll dabei bevorzugt weniger als 1000 ppm betragen, was gemäß der genannten Schrift dadurch erzielt wird, dass die Zugabe an aromatischen Dinitroverbindungen kontrolliert und gesteuert wird.

WO 00/35852 A1 offenbart ein Verfahren zur Herstellung von Aminen durch Hydrierung von Nitroverbindungen, wobei die Hydrierung in einem vertikalen Reaktor, dessen Länge größer ist als sein Durchmesser, mit einer im oberen Bereich des Reaktors angeordneten, nach unten gerichteten Strahldüse, über die die Edukte sowie das Reaktionsgemisch zugeführt werden sowie mit einem Abzug an einer beliebigen Stelle des Reaktors, über den das Reaktionsgemisch in einem äußeren Kreislauf mittels eines Förderorgans der Strahldüse wieder zugeführt wird sowie einer Strömungsumkehr im unteren Bereich des Reaktors durchgeführt wird. Dieses Dokument offenbart nicht die Problematik einer zu hohen Konzentration an Dinitrotoluolen.

DE 198 44 901 C1 offenbart ein Verfahren zur Dosierung von flüssigen Nitroaromaten mit mehr als einer Nitrogruppe in Reaktoren zur Sumpfphasen-Hydrierung. Dazu wird der umzusetzende Nitroaromat in einer oder mehreren Ringleitungen im Kreis gepumpt, wobei diese Ringleitungen ganz oder teilweise im Hydrierreaktor verlaufen und im Reaktorinneren mit Austrittsöffnungen zum Reaktorinneren versehen sind.

WO 2006/089906 A1 offenbart ein Verfahren zur Herstellung von aromatischen Aminen oder aliphatischen Aminoalkoholen, wobei die Konzentration der eingesetzten Nitroverbindungen, bzw. entstehender Nitrosoverbindungen überwacht werden muss, um Sicherheitsrisiken ausschließen zu können. Diese Aufgabe des genannten Dokumentes wird dadurch gelöst, dass eine Online-Überwachung der Konzentration von Nitro- und Nitrosoverbindungen in dem Reaktor erfolgt, beispielsweise durch Messung der Absorption von UV-VIS-Strahlung im Reaktionsgemisch.

WO 03/068724 A1 offenbart ebenfalls ein Verfahren zur Herstellung von Aminen durch Hydrierung der entsprechenden Nitroverbindungen. Des Weiteren wird die Problematik erörtert, dass die Konzentration der Nitroverbindungen nicht zu hoch sein darf, um ein Sicherheitsrisiko zu vermeiden. Die Lösung dieses Dokumentes besteht darin, dass eine bestimmte Menge Wasserstoff in das Reaktionsgemisch eingedüst wird.

Die noch nicht veröffentlichte, europäische Anmeldung mit dem Aktenzeichen EP 10162924.4 betrifft ein Verfahren zur kontinuierlichen Herstellung von Toluylendiamin durch Flüssigphasenhydrierung von Dinitrotoluol mit Wasserstoff in Gegenwart eines suspendierten, Nickel enthaltenden Katalysators, in einem Reaktor, mit einer dem Reaktor nachgeschalteten Produktabtrenneinheit, unter Erhalt eines Produktaustrages aus dem Reaktor umfassend eine Flüssigphase, enthaltend Toluylendiamin und Dinitrotoluol, in der der Nickel enthaltende Katalysator suspendiert ist, wobei die Konzentration an Dinitrotoluol in der Flüssigphase des Produktaustrages aus dem Reaktor im Bereich zwischen dem Reaktor und der nachgeschalteten Produktabtrenneinheit auf einen Wert im Bereich von 1 bis 200 Gew.-ppm, bezogen auf das Gesamtgewicht der Flüssigphase des Produktaustrages aus dem Reaktor, eingestellt wird.

EP 0 634 391 A1 offenbart ein kontinuierliches Verfahren zur Herstellung von aromatischer Di- und Polyaminen durch katalytische Hydrierung der den Aminen entsprechenden Di- und Polynitro-Verbindungen bei hohen Temperaturen und gegebenenfalls gleichzeitiger Wärmeabfuhr aus dem Reaktionsgemisch zur Erzeugung von Dampf mit einem Überdruck von ≥ 2 bar.

Die Verfahren des Standes der Technik sind bezüglich der Vermeidung von Verblockungen aus Edukten, insbesondere durch Nitroaromaten, beispielsweise Dinitrotoluolverbindungen, Katalysator und/oder Produkten, insbesondere den aromatischen Aminen, bevorzugt Toluylendiamin (TDA), zu verbessern.

Unter Verblockung wird im Rahmen der vorliegenden Erfindung eine Ausbildung von festen, nicht löslichen, organische und/oder anorganische Komponenten enthaltenden Ablagerungen innerhalb der einen oder mehreren Zuführungen des Nitroaromaten verstanden. Diese Ablagerungen enthalten beispielsweise Edukt, Produkt, polymere Verbindungen, die aus Edukt und/oder Produkt gebildet werden, Katalysator und/oder gegebenenfalls weitere in der Reaktionslösung vorliegende Substanzen.

Durch entstehende Verblockungen besteht die Möglichkeit der unbemerkten Ausbildung kaum durchströmter Totzonen in denen sich neben Edukt, d. h. Nitroaromat, auch Produkt, polymere Verbindungen, die aus Edukt und/oder Produkt gebildet werden, Katalysator und/oder gegebenenfalls weitere in der Reaktionslösung vorliegende Substanzen befinden können. Ab einer Temperatur von ca. 260 °C kann eine selbstbeschleunigende Zersetzung von Nitroaromat, beispielsweise reinem Dinitrotoluol (DNT), erfolgen. Durch den Kontakt von DNT mit der Produktmischung kann dieser Wert bis unter 100 °C und damit evtl. unter die Reaktionstemperatur absinken. Bei gleichzeitigem Auftreten einer Herabsetzung der Zersetzungstemperatur des DNT durch Produktkontakt sowie langer Verweilzeiten in Totzonen resultiert ein Sicherheitsrisiko für die Durchführung des Verfahrens.

Des Weiteren führt eine Verblockung bestimmter Reaktorteile durch die genannten Verbindungen dazu, dass von Zeit zu Zeit der Reaktor gereinigt werden muss, was zwangsläufig ein Abschalten des bevorzugt kontinuierlich laufenden Reaktors und somit Einbußen bezüglich der Wirtschaftlichkeit und Ausbeute eines solchen Reaktors ergibt.

Aufgabe der vorliegenden Erfindung ist es daher, ein Verfahren zur Herstellung aromatischer Amine durch Hydrierung der entsprechenden Nitroaromaten bereitzustellen, durch das effektiv vermieden werden kann, dass innerhalb der einen oder mehreren Zuführungen des Nitroaromaten Verblockungen aus den eingesetzten Edukten, dem vorliegendem Katalysator sowie gebildeter Produkte entstehen. Diese Verblockungen sollen gering gehalten werden, um so die Explosionsgefahr gering halten zu können. Eine weitere Aufgabe ist es, ein entsprechendes Verfahren bereitzustellen, welches über einen langen Zeitraum kontinuierlich durchgeführt werden kann, ohne dass Störungen am Reaktor, welche durch die oben genannten Verblockungen auftreten können, auftreten. Aufgabe der vorliegenden Erfindung ist es darüber hinaus, ein entsprechendes Verfahren bereitzustellen, bei dem eine schnellst mögliche Untermischung des Nitroaromaten, bevorzugt des Dinitrotoluols, und damit eine bestmögliche Vermeidung lokaler Überkonzentrationen ermöglicht, die eine Katalysatordesaktivierung begünstigen.

Diese Aufgaben werden gelöst durch das erfindungsgemäße Verfahren zur Herstellung von aromatischen Aminen durch Hydrierung von Nitroaromaten mit Wasserstoff in Gegenwart eines suspendierten Katalysators in der flüssigen Phase
- in einem Reaktor (1),
- an dessen einen, bevorzugt oberen, Ende eine Treibstrahldüse (2) angeordnet ist, über die in diesen Bereich des Reaktors (1) das aus dem Reaktorsumpf abgezogene Reaktionsgemisch über eine externe Schlaufe (3) eingedüst wird,
- mit Zuführung des Nitroaromaten an einem, bevorzugt oberen, Ende des Reaktors (1), und
- Zuführung des Wasserstoffs
wobei
- bei bestimmungsgemäßer Füllhöhe des Reaktors weder im Betriebszustand noch bei Abstellung ein direkter räumlicher Kontakt zwischen der Zuführung des Nitroaromaten und der flüssigen Phase, bevorzugt enthaltend das gewünschte aromatische Amin, Wasser und Katalysator, im Reaktor und/oder im Umpumpkreis erfolgen kann, und
- nach einem nicht-bestimmungsgemäßen Kontakt zwischen der Zuführung des Nitroaromaten und der Flüssigphase des Reaktors eine teilweise oder vollständige Verblockung der Zuführung des Nitroaromaten messtechnisch erfasst werden kann.

Für die Hydrierung von Nitroaromaten zu aromatischen Aminen, insbesondere von Dinitrotoluol zu Toluylendiamin, wurde eine Vielzahl von Katalysatoren entwickelt, wobei die Verbesserung von Ausbeute und Selektivität der Umsetzung sowie die Stabilität der Katalysatoren auch bei höheren Reaktionstemperaturen vorrangige Aufgaben bei der Entwicklung neuer Katalysatoren waren.

Als besonders geeignet haben sich Katalysatoren erwiesen, die als Aktivmasse ein oder mehrere Metalle, ausgewählt aus der Gruppe bestehend aus Platin, Palladium, Rhodium, Ruthenium und Mischungen davon und daneben ein oder mehrere weitere Metalle, ausgewählt aus der Gruppe bestehend aus Nickel, Kobalt, Eisen, Zink und Mischungen davon enthalten, und die auf einem inerten Träger aufgebracht ist.

Vorteilhaft sind insbesondere Katalysatoren, die als Aktivmasse Platin und daneben Nickel, oder auch Katalysatoren, die Palladium, Nickel und Eisen oder Katalysatoren, die Palladium, Nickel und Kobalt, enthalten.

Besonders vorteilhaft sind Hydrierkatalysatoren, die Platin und Nickel auf einem Träger in Form einer Legierung mit einem Atomverhältnis von Nickel zu Platin in der Legierung zwischen 30 : 70 und 70 : 30, enthalten.

Legierungen aus Platin und Nickel mit anderen Atomverhältnissen sind für das erfindungsgemäße Verfahren prinzipiell auch brauchbar, sie führen jedoch, insbesondere bei der Durchführung der Hydrierung bei höheren Temperaturen, zu geringen Ausbeuten an aromatischem Amin.

Das Atomverhältnis von Nickel zu Platin, bestimmt mittels EDXS (Energy Dispersive X-Ray Spectroscopy), liegt insbesondere bei zwischen 45 : 55 und 55 : 45.

Der Katalysator enthält zumeist ca. 1 bis 15 nm große, feinkristalline Metallpartikel der Pt-Ni Legierung verteilt auf z. B. Kohlenstoffteilchen. Stellenweise können 1 bis 2 µm große Pt-Ni Teilchenagglomerate oder -aggregate, aber auch vereinzelte reine Ni oder Pt Teilchen auf dem Träger vorkommen. Die Elektronenbeugungslinien der Metallteilchen liegen zwischen denen von Pt und Ni, welche die Legierungsbildung zusätzlich belegt. Die Metallpartikel sind meist polykristallin, und können mit einem hochauflösenden TEM (FEG-TEM: Field Emission Gun-Transmission Electron Microscopy) charakterisiert werden.

Als Träger für die Katalysatoren können die hierfür üblichen und bekannten Materialien eingesetzt werden. Vorzugsweise werden Aktivkohle, Ruß, Graphit oder Metalloxide, bevorzugt hydrothermal stabile Metalloxide, wie z.B. ZrO₂, TiO₂ eingesetzt. Bei Graphit sind die HSAG (High Surface Area Graphite) mit einer Oberfläche von 50 bis 300 m²/g besonders bevorzugt. Besonders bevorzugt sind die physisch oder chemisch aktivierte Aktivkohle oder Ruße, wie Acetylenruß.

Weitere geeignete Katalysatoren enthalten als Aktivkomponente Nickel allein oder zusammen mit mindestens einem Metall der I., V., VI. und/oder VIII. Nebengruppe des Periodensystems. Die Katalysatoren können technisch durch Aufbringen von Nickel und gegebenenfalls mindestens einem der oben genannten zusätzlichen Metalle auf einen geeigneten Träger oder durch Co-Fällung hergestellt werden. Gemäß dieser bevorzugten Ausführungsform der Erfindung weist der Katalysator einen Nickelgehalt zwischen 0,1 und 99 Gew.-%, bevorzugt zwischen 1 und 90 Gew.-%, besonders bevorzugt zwischen 25 und 85 Gew.-% und ganz besonders bevorzugt zwischen 60 und 80 Gew.-%, bezogen auf das Gesamtgewicht des Katalysators auf. Vorzugsweise werden als Metalle der I., II., V., VI. und/oder VIII. Nebengruppe des Periodensystems Palladium, Platin, Rhodium, Eisen, Kobalt, Zink, Chrom, Vanadium, Kupfer, Silber oder ein Gemisch aus zwei oder mehreren davon verwendet. Als Trägermaterialien werden vorzugsweise Aktivkohle, Ruß, Graphit, oder oxidische Trägerkomponenten wie Siliziumdioxid, Siliziumcarbid, Kieselgur, Aluminiumoxid, Magnesiumoxid, Titandioxid, Zirkoniumdioxid und/oder Hafniumdioxid oder ein Gemisch aus zwei oder mehr davon, besonders bevorzugt Zirkoniumdioxid, ZrO₂, HfO₂ und/oder SiO₂, ZrO₂ und/oder SiO₂, ZrO₂, HfO₂, verwendet. Geeignete Katalysatoren dieser Ausführungsform sind z. B. in den Druckschriften EP 1 161 297 A1 und EP 1 165 231 A1 beschrieben.

Als Edukt des erfindungsgemäßen Verfahrens werden Nitroaromaten verwendet. In einer bevorzugten Ausführungsform des erfindungsgemäßen Verfahrens werden Mononitroaromaten oder Dinitroaromaten verwendet, besonders bevorzugt werden erfindungsgemäße Dinitroaromaten zu den entsprechenden aromatischen Diaminen hydriert.

Bevorzugte Mononitroaromaten sind beispielsweise ausgewählt aus der Gruppe bestehend aus Nitrobenzol, Nitrotoluol, beispielsweise 2-Nitrotoluol, 3-Nitrotoluol und/oder 4-Nitrotoluol, und Mischungen davon. Erfindungsgemäß kann Nitrobenzol zu Anilin hydriert werden. Des Weiteren kann Nitrotoluol zu den entsprechenden Toluidinen hydriert werden.

Bevorzugte Dinitroaromaten sind beispielsweise ausgewählt aus der Gruppe bestehend aus Dinitrobenzol, beispielsweise 1,3-Dinitrobenzol, 1,2-Dinitrobenzol und/oder 1,4-Dinitrobenzol, Dinitrotoluol und Mischungen davon. Erfindungsgemäß kann Dinitrobenzol zu Diaminobenzol hydriert werden. Des Weiteren kann Dinitrotoluol zu den entsprechenden Toluylendiamin-Isomeren hydriert werden.

Bevorzugt wird erfindungsgemäß Dinitrotoluol (DNT) eingesetzt. Dinitrotoluol ist dem Fachmann an sich bekannt und kann in verschiedenen Isomeren vorliegen, die im Folgenden abgebildet sind

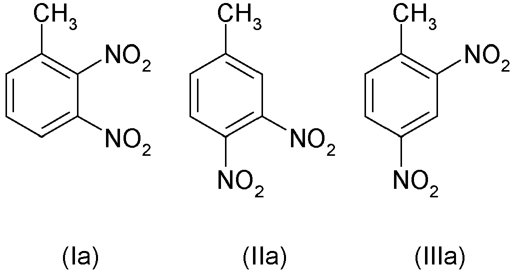

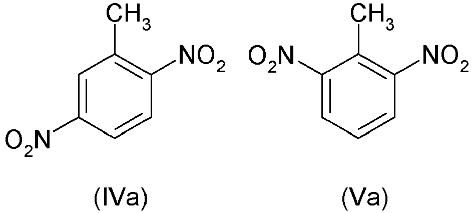

Erfindungsgemäß kann ein einzelnes Isomer ausgewählt aus 2,3-Dinitrotoluol (la), 3,4-Dinitrotoluol (IIa), 2,4-Dinitrotoluol (IIIa), 2,5-Dinitrotoluol (IVa) oder 2,6-Dinitrotoluol (Va) eingesetzt werden. Es ist erfindungsgemäß aber auch möglich, und bevorzugt, eine Mischung enthaltend wenigstens zwei der genannten Isomere einzusetzen, beispielsweise eine Mischung enthaltend 2,4-Dinitrotoluol (IIIa) und 2,6-Dinitrotoluol (Va). In einer bevorzugten Ausführungsfrom des erfindungsgemäßen Verfahrens wird eine Mischung von Dinitrotoluol-Isomeren eingesetzt, wie sie bei der zweifachen Nitrierung von Toluol erhalten wird.

In einer bevorzugten Ausführungsform werden als Produkt des erfindungsgemäßen Verfahrens nach Hydrierung die entsprechenden Toluylendiamine erhalten. Die möglichen Isomere sind im Folgenden abgebildet.

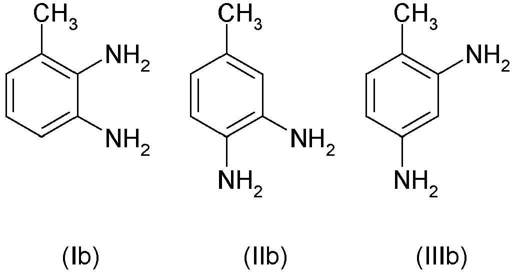

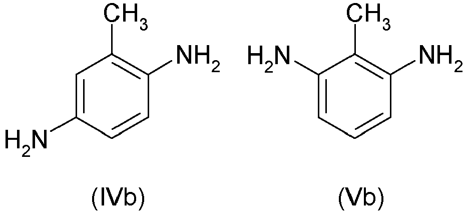

Abhängig davon, welches Isomer bzw. welche Isomere der Dinitrotoluole als Edukte eingesetzt werden, wird als Produkt des erfindungsgemäßen Verfahrens ein entsprechendes Isomer bzw. eine Mischung von mindestens zwei Isomeren des Toluylendiamins erhalten, d. h. erfindungsgemäß bevorzugt findet während der Umsetzung keine Isomerisierung und/oder Hydrierung des aromatischen Systems statt.

Mögliche Produkte sind daher ausgewählt aus Toluylen-2,3-diamin (Ib), Toluylen-3,4-diamin (IIb), Toluylen-2,4-diamin (IIIb), Toluylen-2,5-diamin (IVb) oder Toluylen-2,6-diamin (Vb). Es ist erfindungsgemäß aber auch möglich, und bevorzugt, dass eine Mischung enthaltend wenigstens zwei der genannten Isomere erhalten wird, beispielsweise eine Mischung enthaltend Toluylen-2,4-diamin (IIIb) und Toluylen-2,6-diamin (Vb). Des Weiteren ist es möglich, dass in dem erhaltenen Produktgemisch Verbindungen vorliegen, in denen nur eine Nitrogruppe zu der entsprechenden Aminogruppe hydriert worden ist, so dass noch eine Nitrogruppe im Molekül verblieben ist.

Das Verfahren wird bevorzugt bei Raum-Zeit-Ausbeuten von 100 bis 1000 kg/m3 · h, bevorzugt 150 bis 500 kg/m3 · h, aromatisches Amin durchgeführt. Betrachtet wird dabei das Reaktorvolumen in m³, wobei dieses dem Gesamtvolumen des Reaktors abzüglich dem Volumen von darin vorliegenden Einbauten entspricht.

Das Verfahren kann sowohl unter Zuhilfenahme eines organischen Lösungsmittels, beispielsweise Alkohole wie Methanol, Ethanol, Propanol oder eine Mischung davon, oder ohne Zusatz eines organischen Lösungsmittels durchgeführt werden.

Das erfindungsgemäße Verfahren zur Herstellung von aromatischen Aminen durch Hydrierung von Nitroaromaten mit Wasserstoff in Gegenwart eines suspendierten Katalysators in der flüssigen Phase erfolgt
- in einem Reaktor (1),
- an dessen einen, bevorzugt oberen, Ende eine Treibstrahldüse (2) angeordnet ist, über die in diesen Bereich des Reaktors (1) das aus dem Reaktorsumpf abgezogene Reaktionsgemisch über eine externe Schlaufe (3) eingedüst wird,
- mit Zuführung des Nitroaromaten an einem, bevorzugt oberen, Ende des Reaktors (1), und
- Zuführung des Wasserstoffs
wobei
- bei bestimmungsgemäßer Füllhöhe des Reaktors weder im Betriebszustand noch bei Abstellung ein direkter räumlicher Kontakt zwischen der Zuführung des Nitroaromaten und der flüssigen Phase, bevorzugt enthaltend das gewünschte aromatische Amin, Wasser und Katalysator, im Reaktor und/oder im Umpumpkreis erfolgen kann, und
- nach einem nicht-bestimmungsgemäßen Kontakt zwischen der Zuführung des Nitroaromaten und der Flüssigphase des Reaktors eine teilweise oder vollständige Verblockung der Zuführung des Nitroaromaten messtechnisch erfasst werden kann.

Das erfindungsgemäße Verfahren wird bevorzugt in einem vertikal stehenden Reaktor durchgeführt. Bevorzugt ist die Länge des Reaktors größer als die Breite. Geeignete Abmessungen sind durch den Fachmann leicht zu bestimmen.

Die vorliegende Erfindung betrifft daher bevorzugt das erfindungsgemäße Verfahren, wobei der Reaktor (1) vertikal aufrecht steht.

Das erfindungsgemäße Verfahren wird bevorzugt in einem Reaktor durchgeführt, in dem eine interne und eine äußere Schlaufenbewegung des Reaktionsgemisches statt findet.

Hierzu ist in einem bevorzugt vertikal aufrecht stehenden Reaktor an seinem oberen Ende bevorzugt eine Treibstrahldüse angeordnet, die die innere Schlaufenbewegung antreibt, und zwar bevorzugt, indem über die Treibstrahldüse in den oberen Bereich des Reaktors das aus dem Reaktorsumpf abgezogene Reaktionsgemisch, das über eine externe Schlaufe umgepumpt wird, in den oberen Bereich des Reaktors nach unten gerichtet eingedüst wird.

Das über die Treibstrahldüse eingedüste Reaktionsgemisch strömt bevorzugt durch ein zentrales, in Reaktorlängsrichtung angeordnetes Einsteckrohr und durchströmt dieses von oben nach unten. Das zentrale Einsteckrohr kann als einfaches Rohr ausgestaltet sein. Unterhalb des Einsteckrohrs kehrt sich das Reaktionsgemisch in einer internen Schlaufenbewegung außerhalb des Einsteckrohrs um und strömt erneut nach oben.

Die vorliegende Erfindung betrifft daher bevorzugt das erfindungsgemäße Verfahren, wobei das Reaktionsgemisch, welches über eine externe Schlaufe (3) eingedüst wird, anschließend in ein zentrales Einsteckrohr (4), welches in Reaktorlängsrichtung angeordnet ist, einströmt, dieses der Länge nach, bevorzugt von oben nach unten, durchströmt und in einer internen Schlaufenbewegung außerhalb des Einsteckrohrs (4) erneut nach oben strömt.

Zur Strömungsumkehr ist bevorzugt eine Prallplatte unterhalb des Einsteckrohrs angeordnet.

Durch das bevorzugt konzentrisch vorliegende Einsteckrohr wird in Verbindung mit der bevorzugt vorliegenden Prallplatte die Schlaufenströmung innerhalb des Reaktors, d. h. die interne Schlaufenströmung, stabilisiert.

Durch den Treibstrahl wird Gas aus dem Gasraum in die Flüssigkeit in Form von Gasblasen bis zur Prallplatte mitgerissen. Diese Gasblasen steigen im Ringraum, d. h. zwischen Einsteckrohr und Reaktorwand, des Reaktors wieder auf. Durch diese interne Kreisgasfahrweise wird eine große Gas/Flüssig-Phasengrenzfläche zur Verfügung gestellt.

Im Reaktorinnenraum ist zur Wärmeabführung bevorzugt ein Wärmetauscher angeordnet, durch den Kühlwasser strömt, das einen Teil der Reaktionswärme aufnimmt.

Die vorliegende Erfindung betrifft daher bevorzugt das erfindungsgemäße Verfahren, wobei ein Wärmetauscher (6) im Innenraum des Reaktors (1) vorliegt, durch den Kühlwasser strömt, und hierbei einen Teil der Reaktionswärme aufnimmt.

Der im Reaktorinnenraum angeordnete Wärmetauscher ist bevorzugt ein Fieldrohr-Wärmetauscher.

In einer Ausführungsform ist der im Innenraum des Reaktors angeordnete Wärmetauscher ein Rohrschlangen-Wärmetauscher.

In einer weiteren Ausführungsform ist der im Innenraum des Reaktors angeordnete Wärmetauscher ein Rohrbündel-Wärmetauscher.

In einer weiteren Ausführungsform ist der im Innenraum des Reaktors angeordnete Wärmetauscher ein Platten-Wärmetauscher.

In einer bevorzugten Ausführungsform des erfindungsgemäßen Verfahrens wird zusätzlich zu dem im Innenraum des Reaktors angeordneten Wärmetauscher ein weiterer Wärmetauscher in der externen Schlaufe eingesetzt. Dieser bevorzugt vorliegende zweite Wärmetauscher dient bevorzugt dazu, den restlichen Teil der Reaktionswärme, der über den internen Wärmetauscher nicht abgeführt werden kann, abzuführen. Bevorzugt kommt dabei ein Rohrbündel-Wärmetauscher zum Einsatz.

Die vorliegende Erfindung betrifft daher bevorzugt das erfindungsgemäße Verfahren, wobei zusätzlich zu dem im Innenraum des Reaktors (1) angeordneten Wärmetauscher (6) ein weiterer Wärmetauscher (W) in der externen Schlaufe eingesetzt wird.

Aus der freiwerdenden Reaktionswärme kann Dampf sowohl in dem internen als auch dem externen Wärmetauscher auf zwei Arten erzeugt werden. Zum einen durch Verdampfung eines Teils des Kühlwassers in den Kühlrohren (Direktdampferzeugung) oder durch Erhitzen des Kühlwassers bei einem Druck, der oberhalb des Druckes des zu erzeugenden Dampfes liegt, und anschließende Entspannung auf das Druckniveau des zu erzeugenden Dampfes (Entspannungsverdampfung). Bei dieser Entspannung verdampft ein Teil des Kühlwassers, und das Dampf/Wasser-Gemisch kühlt sich auf die dem Druck des Dampfes entsprechende Siedetemperatur ab.

Beide Arten der Verdampfung können sowohl im internen und als auch externen Wärmetauscher angewendet werden. Ebenso ist eine Kombination beider Verdampfungsarten möglich, das heißt Direktverdampfung im internen Wärmetauscher und Entspannungsverdampfung im externen Wärmetauscher oder umgekehrt.

Abhängig z.B. von der Reaktionstemperatur kann aber auch auf die Dampferzeugung verzichtet und die Wärme durch Rückkühlwasser aufgenommen werden.

Nitroaromaten werden bevorzugt am oberen Ende des Reaktors zugeführt, bevorzugt in die Gasphase oberhalb des Flüssigkeitsspegels im Reaktor.

In einer bevorzugten Ausführungsform des erfindungsgemäßen Verfahrens wird die Zuführung des Nitroaromaten durch eine Zulaufleitung und/oder eine Dosiereinrichtung realisiert.

Für das erfindungsgemäße Verfahren ist es erfindungswesentlich, das bei bestimmungsgemäßer Füllhöhe des Reaktors weder im Betriebszustand noch bei Abstellung ein direkter räumlicher Kontakt zwischen der Zuführung des Nitroaromaten und der flüssigen Phase, bevorzugt enthaltend das gewünschte aromatische Amin, Wasser und Katalysator, im Reaktor und/oder im Umpumpkreis erfolgen kann.

Unter einer bestimmungsgemäßen Füllhöhe wird hierbei eine Füllhöhe mit einem Flüssigkeitsspiegel zwischen der Oberkante eines bevorzugt vorliegenden Einsteckrohrs und der Austrittsöffnung der Treibstrahldüse verstanden. Bevorzugt befindet sich der Flüssigkeitsspiegel näher an der Austrittsöffnung der Treibstrahldüse als an der Oberkante des Einsteckrohrs.

In einer bevorzugten Ausführungsform des erfindungsgemäßen Verfahrens wird die Zuführung des Nitroaromaten durch eine Zulaufleitung sowie eine Dosiereinrichtung ohne geometrische Möglichkeit einer Totraumbildung realisiert. Entsprechende Einheiten bzw. Vorrichtungen sind dem Fachmann an sich bekannt. Erfindungsgemäß essentiell ist es dabei, dass bei bestimmungsgemäßer Füllhöhe des Reaktors weder im Betriebszustand noch bei Abstellung ein direkter räumlicher Kontakt zwischen der Zuführung des Nitroaromaten und der flüssigen Phase, bevorzugt enthaltend das aromatische Amin, d. h. das entsprechende Reaktionsprodukt, Wasser und Katalysator, im Reaktor und/oder im Umpumpkreis erfolgen kann. Dies wird erfindungsgemäß beispielsweise dadurch realisiert, dass der Abstand zwischen der Zuführung des Nitroaromaten und der flüssigen Phase beispielsweise 0,01 bis 3 m, bevorzugt 0,05 bis 1,0 m beträgt.

In einer weiter bevorzugten Ausführungsform des erfindungsgemäßen Verfahrens wird die Zuführung des Nitroaromaten über eine oder mehrere unabhängige Rohrleitungen realisiert, deren teilweise oder vollständige Verblockung messtechnisch erfasst werden kann.

Entsprechende Rohrleitungen sind dem Fachmann an sich bekannt. Die bevorzugt eingesetzten Rohrleitungen sind beispielsweise aus Metall, beispielsweise Schwarzstahl oder Edelstahl, gefertigt. Die Rohrleitungen weisen bevorzugt Durchmesser auf, die sich nach den Mengen an Nitroaromaten, welche zugeführt werden, bemessen.

Im Allgemeinen können die bevorzugt eingesetzten Rohrleitungen jeden geeigneten Querschnitt aufweisen.

In einer bevorzugten Ausführungsform des erfindungsgemäßen Verfahrens weist der Austrittsquerschnitt der Zuführung des Nitroaromaten an den Enden der einen oder mehreren unabhängigen Rohrleitung(en) eine Verengung oder eine von der rotationssymmetrischen Form abweichende Gestalt auf. Der austretende Nitroaromat-Strahl kann laminares oder turbulentes Verhalten aufweisen.

Es ist erfindungsgemäß auch günstig, wenn bei der Einmischung des frisch zugeführten Nitroaromaten in die Reaktionsmischung signifikante lokale Überkonzentrationen vermieden werden. Erhöhte (lokale) Konzentrationen von Nitroaromaten führen zu verstärkter Nebenproduktbildung und Deaktivierung des suspendierten Katalysators. Daraus resultieren ein Ausbeuteverlust und damit ein höherer Aufwand zur Produktaufarbeitung einerseits und erhöhte Katalysatorkosten sowie Kapazitätseinschränkungen andererseits. Außerdem können Sicherheitsprobleme auftreten.

Durch den Treibstrahl des äußeren Wälzkreises wird im Innern des Reaktors bevorzugt eine zirkulierende Strömung hervorgerufen. Der Abwärtsstrombereich dieser Zirkulationsströmung ist durch die Querschnittsfläche des bevorzugt vorliegenden Einsteckrohres parallel zur Reaktorachse festgelegt. Der Aufwärtsströmbereich wird durch die Querschnittsfläche des Ringspalts zwischen dem bevorzugt vorliegenden Einsteckrohr und Reaktormantel festgelegt.

Der Treibstrahl des äußeren Wälzkreises breitet sich im Reaktionsmedium bevorzugt als turbulenter Freistrahl aus. Am Umfang eines turbulenten Freistrahls findet durch Impulseintrag eine Beschleunigung und Einmischung des umgebenden Fluids statt.

Bei Aufbringung von Nitroaromaten auf die Flüssigkeitsoberfläche im Reaktorinneren wird die Wirksamkeit der Einmischung demzufolge mit zunehmender Nähe zum Auftreffbereich des Treibstrahls auf die Flüssigkeitsoberfläche verbessert. Die Wirksamkeit der Einmischung bestimmt die räumliche Ausdehnung der Einmischzone sowie der Höhe der darin stationär auftretenden Nitroaromat-Überkonzentration im Vergleich zum Gesamtreaktorvolumen.

Daher betrifft die vorliegende Erfindung bevorzugt das erfindungsgemäße Verfahren, wobei ein laminarer oder turbulenter Nitroaromaten-Strahl aus einer oder mehreren der Zuführungen des Nitroaromaten im Bereich des Umfangs des auf die Flüssigkeitsoberfläche treffenden Treibstrahls der Düse des äußeren Umlaufs auf die Oberfläche der flüssigen Phase aufgegeben wird.

In der erfindungsgemäß bevorzugten Ausführungsform, in der der erfindungsgemäß eingesetzte Reaktor ein zentrales Einsteckrohr (4), welches in Reaktorlängsrichtung angeordnet ist, aufweist, wird in dem erfindungsgemäßen Verfahren ein laminarer oder turbulenter Nitroaromaten-Strahl aus einer oder mehreren der Zuführungen des Nitroaromaten im Bereich der auf die Flüssigkeitsoberfläche projizierten Querschnittsfläche des Einsteckrohres, bevorzugt möglichst nahe am Treibstrahl der Düse des äußeren Umlaufs, besonders bevorzugt direkt am Umfang des Treibstrahls der Düse des äußeren Umlaufs auf die Oberfläche der flüssigen Phase, aufgegeben.

In einer weiteren bevorzugten Ausführungsform des erfindungsgemäßen Verfahren sind die Austrittsöffnungen der einen oder mehreren Zuführungen des Nitroaromaten zur Erzeugung eines radialen Impulses um bis zu 90° in Richtung der Symmetrieachse des Reaktors geneigt. In dieser Ausführungsform sind die Austrittsöffnungen um beispielsweise 10 bis 80°, bevorzugt 10 bis 45° in Richtung der Symmetrieachse des Reaktors geneigt, d. h., dass die Austrittsöffnungen der Zuführungen bevorzugt in Richtung der Mitte des Reaktors geneigt sind. Dadurch trifft der Nitroaromat-Strahl nicht im rechten Winkel auf die flüssige Phase auf, sondern in einem spitzeren Winkel, so dass ein radialer Impuls in Richtung des mischungsrelevanten Treibstrahls resultiert. Dies trägt dazu bei, dass eine schnelle und gute Durchmischung erreicht wird.

Das erfindungsgemäße Verfahren weist des Weiteren das wesentliche Merkmal auf, dass nach einem nicht-bestimmungsgemäßen Kontakt zwischen der Zuführung des Nitroaromaten und der Flüssigphase des Reaktors eine teilweise oder vollständige Verblockung der Zuführung des Nitroaromaten messtechnisch erfasst werden kann.

Messtechnische Verfahren und Vorrichtungen zur Erfassung einer entsprechenden Verblockung sind dem Fachmann an sich bekannt, beispielsweise Manometer oder Durchflussmesser. Beispielsweise kann eine Verblockung dadurch detektiert werden, dass ein Stoffstrom bei konstanter Druckdifferenz abnimmt. Des Weiteren kann eine Verblockung beispielsweise durch einen Anstieg des Druckes an der entsprechenden Stelle bei konstantem Stoffstrom detektiert werden.

Die Erfinder haben erkannt, dass es vorteilhaft ist, den Nitroaromaten, bevorzugt das Dinitrotoluol, in die Gasphase oberhalb des Flüssigkeitsspiegels im Reaktor einzuleiten; hierdurch wird verhindert, dass Reaktionsprodukt zurück in die Zuführleitung für Nitroaromaten, bevorzugt Dinitrotoluol, strömt und dass es dadurch zur Zersetzung oder Explosion des Nitroaromaten führt. Reines Dinitrotoluol hat beispielsweise eine Zersetzungstemperatur von etwa 260 °C, die Zersetzungstemperatur sinkt jedoch drastisch sobald Toluylendiamin zugemischt wird bis auf unter 100 °C.

Daher ist es auch vorteilhaft, die Zuführleitung des Nitroaromaten bei Unterbrechungen der Produktion oder Abstellungen der Anlage mit heißem Wasser frei zu spülen. Dies gilt insbesondere nach einem nicht bestimmungsgemäßen Zustand, beispielsweise einer Überfüllung des Reaktors.

Die Erfinder haben erkannt, dass es bevorzugt ist, dass sich im Reaktor weniger als 1000 ppm Nitroaromat akkumuliert. Hierzu ist es sinnvoll, im Bereich zwischen dem Reaktor und der nachgeschalteten Produktabtrenneinheit eine Nitroaromat-Konzentration zwischen 1 und 200 ppm sicherzustellen. Dies dient gleichsam einer abschließenden Kontrolle der Misch- und Reaktionsprozesse in den vorgelagerten Reaktoren.

Der für die Hydrierung notwendige Wasserstoff wird am unteren Ende des Reaktors, bevorzugt über einen Ringverteiler, zugeführt.

Ein weiteres erfindungswesentliches Merkmal des erfindungsgemäßen Verfahrens ist, dass die Konzentration an Wasserstoff im Reaktionsgemisch, das aus dem Reaktorsumpf in die externe Schlaufe strömt 0,1 Vol.-%, bevorzugt 3 Vol.-%, nicht unterschreitet. Durch diese erfindungsgemäße Mindestkonzentration im Reaktionsgemisch wird beispielsweise gewährleistet, dass ein gewünschter hoher Umsatz des Nitroaromaten, bevorzugt des Dinitrotoluols, und somit eine niedrige Nitroaromat-Konzentration, bevorzugt Dinitrotoluol-Konzentration, auch im Bereich der externen Umlaufströmung erreicht werden.

In einer weiteren bevorzugten Ausführungsform des erfindungsgemäßen Verfahrens wird dem in der externen Schlaufe strömenden Reaktionsgemisch zusätzlicher Wasserstoff, bevorzugt möglichst nahe am Reaktor zugeführt, um die Mindestkonzentration des Wasserstoffs in der externen Schlaufe sicherzustellen.

In einer weiteren bevorzugten Ausführungsform des erfindungsgemäßen Verfahrens wird dem Reaktor an beliebiger Stelle Wasserstoff zugeführt und der Reaktordurchmesser oder die Abströmgeschwindigkeit des Reaktionsgemisches aus dem Reaktorsumpf werden so ausgelegt, dass die Mindestkonzentration des Wasserstoffs in der externen Schlaufe sichergestellt ist. Die Abströmgeschwindigkeit des Reaktionsgemisches kann durch dem Fachmann bekannte Maßnahmen, beispielsweise eine geeignete Gestaltung des Reaktordurchmessers beeinflusst werden.

Um den externen Wälzkreis antreiben zu können, wird bevorzugt eine Pumpe installiert, die neben Flüssigkeit auch Gas und suspendierten Feststoff fördern kann. Dabei sollten bis zu 20 Vol.-% Gas und bis zu 20 Gew.-%. suspendierter Feststoff pumpbar sein.

Die Reaktionstemperatur des erfindungsgemäßen Verfahrens beträgt im Allgemeinen 80 bis 200 °C, bevorzugt 100 bis 180 °C, besonders bevorzugt 110 bis 140 °C.

Der Reaktionsdruck des erfindungsgemäßen Verfahrens beträgt im Allgemeinen 10 bis 50 bar absolut, bevorzugt 10 bis 30 bar absolut, besonders bevorzugt 20 bis 30 bar absolut.

Ganz besonders bevorzugt betrifft die vorliegende Erfindung das erfindungsgemäße Verfahren zur Herstellung von Toluylendiamin (TDA) durch Hydrierung von Dinitrotoluol (DNT) mit Wasserstoff in Gegenwart eines suspendierten Katalysators in der flüssigen Phase
- in einem vertikal aufrecht stehenden Reaktor (1),
- an dessen oberem Ende eine Treibstrahldüse (2) angeordnet ist, über die in den oberen Bereich des Reaktors (1) das aus dem Reaktorsumpf abgezogene Reaktionsgemisch über eine externe Schlaufe (3) eingedüst wird, das anschließend in ein zentrales Einsteckrohr (4), welches in Reaktorlängsrichtung angeordnet ist, einströmt, dieses von oben nach unten durchströmt und in einer internen Schlaufenbewegung außerhalb des Einsteckrohrs (4) erneut nach oben strömt,
- mit einem Wärmetauscher (6) im Innenraum des Reaktors (1), durch den Kühlwasser strömt, und hierbei einen Teil der Reaktionswärme aufnimmt,
- mit Zuführung des DNT am oberen Endes des Reaktors (1), und
- Zuführung des Wasserstoffs am unteren Ende des Reaktors (1),
wobei
- bei bestimmungsgemäßer Füllhöhe des Reaktors weder im Betriebszustand noch bei Abstellung ein direkter räumlicher Kontakt zwischen der Zuführung des DNT und der flüssigen Phase, bevorzugt enthaltend TDA, Wasser und Katalysator, im Reaktor und/oder im Umpumpkreis erfolgen kann,
- nach einem nicht-bestimmungsgemäßen Kontakt zwischen der Zuführung des DNT und der Flüssigphase des Reaktors eine teilweise oder vollständige Verblockung der genannten Elemente messtechnisch erfasst werden kann, und
die Konzentration an Wasserstoff im Reaktionsgemisch, das aus dem Reaktorsumpf in die externe Schlaufe strömt 0,1 Vol.-%, bevorzugt 3 Vol.-%, nicht unterschreitet.

Die vorliegende Erfindung betrifft auch die Verwendung einer Vorrichtung zur Durchführung des erfindungsgemäßen Verfahrens, mindestens umfassend
- einen Reaktor (1)
- an dessen einen, bevorzugt oberem, Ende eine Treibstrahldüse (2) angeordnet ist, über die in diesen Bereich des Reaktors (1) das aus dem Reaktorsumpf abgezogene Reaktionsgemisch über eine externe Schlaufe (3) eingedüst werden kann,
- eine Zuführung für den Nitroaromaten, an einem oberen Ende des Reaktors (1), so dass bei bestimmungsgemäßer Füllhöhe des Reaktors weder im Betriebszustand noch bei Abstellung ein direkter räumlicher Kontakt zwischen der Zuführung des Nitroaromaten und der flüssigen Phase im Reaktor und/oder im Umpumpkreis erfolgen kann,
- eine Zuführung für den Wasserstoff und
- eine Messeinrichtung zur Bestimmung einer teilweisen oder vollständigen Verblockung der Zuführung des Nitroaromaten nach einem nichtbestimmungsgemäßen Kontakt zwischen der Zuführung des Nitroaromaten und der Flüssigphase des Reaktors.

Bevorzugt betrifft die vorliegende Erfindung die erfindungsgemäße Verwendung, wobei der Reaktor vertikal aufrecht steht.

Weiter bevorzugt betrifft die vorliegende Erfindung die erfindungsgemäße Verwendung, wobei ein zentrales Einsteckrohr (4) in Reaktorlängsrichtung angeordnet ist.

Weiter bevorzugt betrifft die vorliegende Erfindung die erfindungsgemäße Verwendung, wobei ein Wärmetauscher (6) im Innenraum des Reaktors (1) vorliegt, durch den Kühlwasser strömt, und hierbei einen Teil der Reaktionswärme aufnimmt.

Weiter bevorzugt betrifft die vorliegende Erfindung die erfindungsgemäße Vorrichtung, wobei die Zuführung des Wasserstoffs am unteren Ende des Reaktors (1) erfolgt.

Bezüglich der einzelnen Komponenten und der bevorzugten Ausführungsformen der erfindungsgemäß verwendeten Vorrichtung gilt das bezüglich des erfindungsgemäßen Verfahrens Gesagte.

### Figuren

### Figur 1A

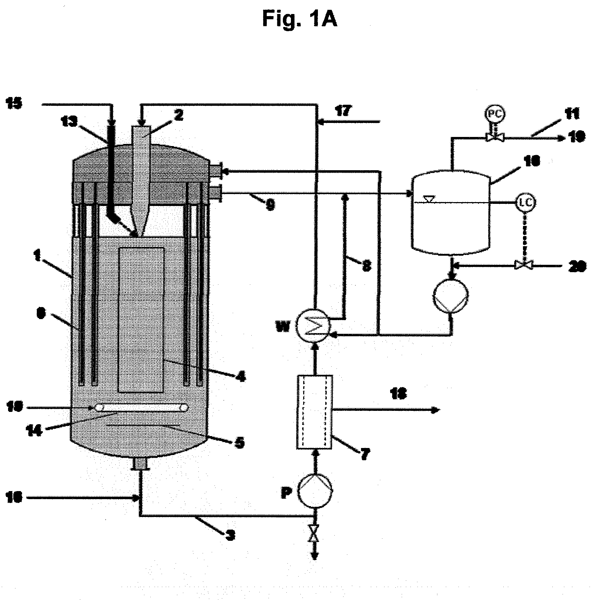

Figur 1A zeigt die schematische Darstellung einer Anlage zur Durchführung des erfindungsgemäßen Verfahrens.

Der Reaktor 1 ist mit einer in seinem oberen Bereich, nach unten ausgerichteten Treibstrahldüse 2 ausgestattet, über die das Reaktionsgemisch über eine externe Schlaufe 3 in den Reaktor eingedüst wird. Unterhalb der Treibstrahldüse 2 ist ein zentrales, in Reaktorlängsrichtung angeordnetes Einsteckrohr 4 angeordnet und unterhalb des Einsteckrohrs 4 eine Prallplatte 5. Im Innenraum des Reaktors 1 befindet sich ein Fieldrohr-Wärmetauscher 6. Dinitrotoluol, DNT, wird in der in der Figur dargestellten bevorzugten Ausführungsvariante über ein einzelnes an seinem Ende in Richtung der Reaktormitte abgewinkeltes Einleitrohr 13 in den Gasraum oberhalb des Flüssigkeitsspiegels in den Reaktor 1 zugeführt.

Wasserstoff H₂, wird in den unteren Bereich des Reaktors 1, in der in der Figur dargestellten bevorzugten Ausführungsvariante, über einen Ringverteiler 14, und zusätzlich in die externe Schlaufe 3, in der Nähe des Abzugs des Reaktionsgemisches aus dem Reaktorsumpf, eingedüst. Das Reaktionsgemisch wird in der externen Schlaufe über eine Pumpe P, die so ausgelegt ist, dass sie bis zu 20 Vol.-% Gas fördern kann, in der in der Figur dargestellten bevorzugten Ausführungsform über einen dem Produktaustrag dienenden Querstromfilter 7 und einen in der externen Schlaufe angeordneten Wärmetauscher W geleitet, der bevorzugt als Rohrbündelwärmetauscher ausgebildet ist. Aus dem in der externen Schlaufe angeordneten Wärmetauscher W wird Dampf über Leitung 8 abgezogen und mit Dampf über Leitung 9 aus den Fieldrohren, die mit Wasser, H₂O, gespeist werden, vereinigt, einem Abscheider 10 zugeführt und als Dampf mit 4 bar Überdruck über Leitung 11 abgezogen.

### Figur 1B

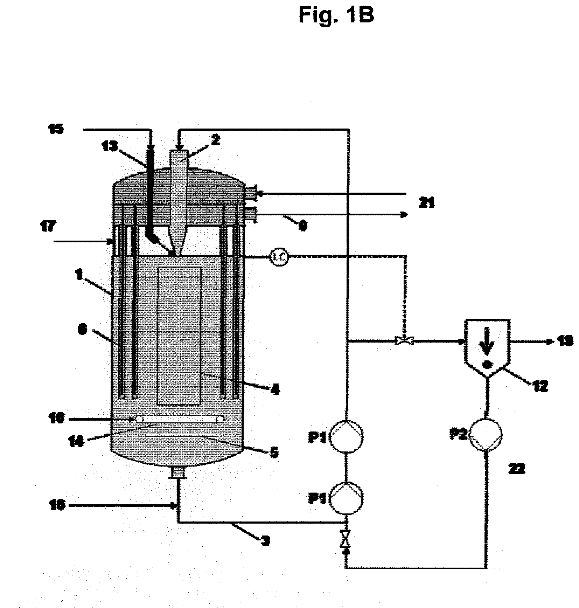

Figur 1B zeigt die schematische Darstellung einer Anlage zur alternativen Durchführung des erfindungsgemäßen Verfahrens.

Der Reaktor 1 ist mit einer in seinem oberen Bereich, nach unten ausgerichteten Treibstrahldüse 2 ausgestattet, über die das Reaktionsgemisch über eine externe Schlaufe 3 in den Reaktor eingedüst wird. Unterhalb der Treibstrahldüse 2 ist ein zentrales, in Reaktorlängsrichtung angeordnetes Einsteckrohr 4 angeordnet und unterhalb des Einsteckrohrs 4 eine Prallplatte 5. Im Innenraum des Reaktors 1 befindet sich ein mit Sekundärkühlwasser 9 gekühlter Fieldrohr-Wärmetauscher 6. Dinitrotoluol, DNT, wird in der in der Figur dargestellten bevorzugten Ausführungsvariante über ein einzelnes an seinem Ende in Richtung der Reaktormitte abgewinkeltes Einleitrohr 13 in den Gasraum oberhalb des Flüssigkeitsspiegels in den Reaktor 1 zugeführt.

Wasserstoff, H₂, wird in den unteren Bereich des Reaktors 1, in der in der Figur dargestellten bevorzugten Ausführungsvariante, über einen Ringverteiler 14, und zusätzlich in die externe Schlaufe 3, in der Nähe des Abzugs des Reaktionsgemisches aus dem Reaktorsumpf, eingedüst. Das Reaktionsgemisch wird über die externe Schlaufe und zwei in Reihe geschaltete Kreislaufpumpen P1, die so ausgelegt sind, dass sie bis zu 20 Vol.-% Gas fördern können, in der in der Figur dargestellten alternativen bevorzugten Ausführungsform zurück auf die Treibstrahldüse des Reaktors geführt. Auf der Druckseite der Kreislaufpumpen wird über eine Standhaltung ein Teilstrom zur Katalysatorabtrennung beispielsweise in einen Schwerkraftabscheider 12 geleitet. Aus diesem wird neben dem Katalysator-freien oder Katalysator-armen Produktstrom eine mit Katalysator angereicherte Suspension über eine weitere Pumpe P2 zurück auf die Saugseite der Kreislaufpumpen gefördert.

Die weiteren im Text nicht genannten Bezugszeichen in den Figuren 1A und 1B haben die folgenden Bedeutungen:
- 15: DNT-Zuführung
- 16: H₂-Zuführung
- 17: Katalysator-Zuführung
- 18: Produktauslass
- 19: Dampf
- 20: Wasser
- 21: Kühlwasser
- 22: mit Katalysator angereicherte Suspension

### Figur 2

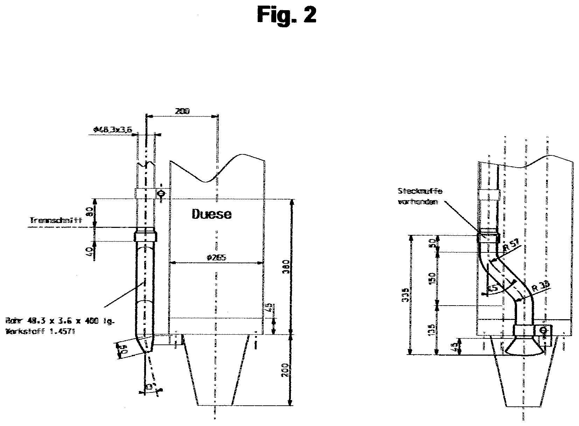

Figur 2 zeigt eine bevorzugte Ausführungsform der DNT-Zuführung. Diese ist als Edelstahlrohr ausgebildet, dessen Mündung gequetscht und zur Treibstrahldüse abgewinkelt ist.

### Beispiele

### Beispiel 1 (erfindungsgemäß)

Es wurde ein zylinderförmiger Schlaufenreaktor mit einem durch zwei in Reihe geschaltete Kreiselpumpen angetriebenen, in einer zentral am Reaktorkopf angeordneten Treibstrahldüse mündenden externen Kreislauf, einem konzentrischen Einsteckrohr sowie einer Prallplatte im unteren Reaktorteil zur Umlenkung der Schlaufenströmung (interner Kreislauf) eingesetzt. Bezüglich dieser Merkmale siehe auch WO 2000/35852. Das Reaktionsvolumen des Reaktors betrug rund 14 m³. Der Reaktor war zur Abfuhr der Reaktionswärme mit einem Rohrbündel aus parallel geschalteten Fieldrohren versehen. Die Menge des in die Fieldrohre eingespeisten Kühlwassers wurde so eingestellt, dass die Temperatur im Reaktor bei ca. 120 °C gehalten wird. Zur Aufrechterhaltung der Schlaufenströmung wurde im externen Produktkreislauf ein Volumenstrom von 580 m³/h umgewälzt, wodurch sich über die Treibstrahldüse ein Druckverlust von ca. 2,3 bar einstellt. Der Reaktor enthielt ca. 12 m³ eines flüssigen Hydrierbads.

Dieses bestand im Wesentlichen aus einer Mischung von Toluylendiamin (TDA) und Wasser im Massenverhältnis von 0,58 : 0,42, in welcher ca. 4 Gew.-% eines auf SiO₂ und ZrO₂ geträgerten metallischen Ni-Katalysators (hergestellt nach Besp. 2 der EP 1 161 297 und mittels einer Rührwerksmühle zerkleinert, hierbei sind 10 Vol.-% des Katalysators Partikel mit einem Durchmesser ≤ ca. 5 µm, 50 Vol% sind ≤ ca. 10 µm, und 90 Vol% ≤ ca. 15 µm, gemessen mittels Laserbeugung (Malvern Mastersizer S) nach Aufrühren in Wasser) suspendiert und außerdem Wasserstoff gelöst waren. Der Flüssigkeitsspiegel der flüssigen Phase befand sich hierbei knapp unterhalb der Mündung der Treibstrahldüse. Darüber befanden sich ca. 2 m³ einer Gasatmosphäre, deren Wasserstoffgehalt durch kontinuierliche Ausschleusung eines geringen Abgasstroms auf 90 bis 95 Vol.-% (neben Inertgasen wie z.B. N₂) eingestellt wurde.

Mit einer Membrankolbendosierpumpe wurden in den Gasraum des Reaktors 7,9 t/h geschmolzenes und auf ca. 80 °C temperiertes Dinitrotoluol (DNT, bestehend aus einer Mischung der 2,4- und 2,6-DNT-Isomeren im Verhältnis von ca. 80 : 20, sowie ca. 5 % der übrigen DNT-Isomeren und Spuren von Mononitrotoluol) eingedüst. Die Zuführung in den Reaktor erfolgte hierbei über das in Fig. 2 abgebildete Edelstahlrohr, dessen Mündung gequetscht und zur Treibstrahldüse hin um einen Winkel von 13° abgewinkelt war. Der durch die Leitung fliesende Volumenstrom und der Druckverlust über die Leitung wurden kontinuierlich überwacht.

Durch gleichzeitiges Einleiten von ca. 0,54 t/h Wasserstoff (verdünnt mit ca. 2 kg/h N₂) wurde im Reaktor ein Druck von 25 barü eingestellt. 95% des Wasserstoffs wurden über einen Düsenring oberhalb der Prallplatte und 5% am Reaktorausgang jeweils ins Hydrierbad dosiert. Die Reaktion verlief unter weitgehend isothermen Bedingungen: Im gesamten Reaktor bewegte sich die Reaktionstemperatur konstant zwischen 117 und 127 °C. Zusätzlich wurden über eine Membranpumpe ebenfalls kontinuierlich 625 kg/h einer Suspension des oben genannten Katalysators in Wasser (teilweise im Aufarbeitungsteil aus dem Hydrierprodukt abgetrennt) dosiert. Die Menge des enthaltenen Katalysators in dieser Suspension wurde hierbei zur Einstellung der DNT-Konzentration gezielt zwischen 0 und 5 kg/h variiert und betrug im Mittel etwa 1,4 kg/h.

Um den Flüssigkeitsstand im Reaktor konstant zu halten, wurde dem externen Produktkreislauf auf der Druckseite der 2. Kreiselpumpe kontinuierlich eine entsprechende Menge an Hydrierprodukt entnommen und in einen Lamellenklärer mit ca. 50 m³ Flüssigkeits- und ca. 10 m³ Gasvolumen geleitet. In dessen unterem Bereich konnte sich der Katalysator aufkonzentrieren. 18 m³/h einer entsprechend eingedickten Suspension wurden dann auf die Saugseite der 1. Kreiselpumpe zurückgeführt. Gleichzeitig wurden dem Lamellenklären über einen Überlauf ca. 9,1 t/h Hydrierprodukt entnommen. Dieses enthielt ca. 5,2 t/h TDA (mit einer Isomerenverteilung entsprechend der des eingesetzten DNT), ca. 0,1 t/h Leicht- und Schwersieder (im Verhältnis von etwa 20 : 80) sowie ca. 3,8 t/h Wasser und bis zu etwa 1 kg/h Katalysator (v.a. Feinanteil). Das Hydrierprodukt gelangte über eine Druckreduzierung ebenso wie die Hydrierprodukte anderer Reaktoren in einen gemeinsamen Zwischenbehälter und wurde von diesem kontinuierlich der destillativen Aufarbeitung zugeführt. Die produktberührenden Teile waren teilweise aus Schwarzstahl (St37) und teilweise aus Edelstahl (1.4571) gefertigt.

Zur Bestimmung des Gehalts an DNT und Aminonitrotoluolen (teilhydrierte Zwischenprodukte) im Hydrierbad wurden im Abstand von maximal 4 h aus der Leitung vom externen Produktkreislauf des Schlaufenreaktors zum Lamellenklärer Suspensionsproben entnommen. Diese wurden durch Filtration vom suspendierten Feststoff befreit und mittels Polarographie die Konzentration der enthaltenen Nitroverbindungen bestimmt. Durch Anpassung der Katalysatorkonzentration in der dem Reaktor zugeführten wässrigen Suspension (s.o.) wurde die DNT-Konzentration zwischen 3 und 30 ppm (im Mittel: 10 ppm) und die Aminonitrotoulol-Konzentration zwischen 1 und 200 ppm (im Mittel: 3 ppm) eingestellt.

Der Reaktor wurde unter den genannten Bedingungen über 2 Monate hinweg ohne nennenswerte Unterbrechungen betrieben. In dieser Zeit nahm die mittels Röntgenpulver-diffraktometrie bestimmte Kristallitgröße des jeweils von den obigen Suspensionsproben abfiltrierten Ni-Katalysators durch Sinterung von 10 nm auf 13 nm zu.

Vor und nach der DNT-Dosierung wurde die DNT-Leitung mit ca. 50 I VE-Wasser in Richtung Reaktor gespült.

Ein Blockieren der Düse wurde weder während dieses kontinuierlichen Betriebs noch zu einem anderen Zeitpunkt (z.B. bei Befüll- und Entleerschritten) detektiert. Auch bei einer Routineinspektion waren keine Verblockungen oder Ablagerungen erkennbar.

### Vergleichsbeispiel 1

Die Umsetzung wurde wie in Beispiel 1 beschrieben ebenfalls über 2 Monate hinweg durchgeführt. Allerdings wurde statt der dort beschriebenen DNT-Einrohrdüse ein DNT-Ringverteiler mit sechs im Wesentlichen senkrecht nach unten gerichteten Öffnungen benutzt. Hierbei konnten bei vergleichbaren DNT- und ANT-Konzentrationen sowie einer vergleichbaren Menge an zudosiertem Katalysator nur 7,5 t/h DNT umgesetzt werden. Entsprechend wurden ca. 0,50 t/h Wasserstoff zugeführt. Die Menge des in der zugeführten wässrigen Suspension enthaltenen Katalysators musste trotz dieser verringerten umzusetzenden DNT-Menge zur Einstellung der DNT- (Mittelwert: 11 ppm) und Aminonitrotoulol-Konzentration (Mittelwert: 1 ppm) in den im Beispiel 1 genannten Bereichen auf im Mittel ca. 1,6 kg/h eingestellt werden. Es wurden ca. 8,6 t/h Hydrierprodukt entnommen. Dieses enthielt ca. 4,9 t/h TDA (mit einer Isomerenverteilung entsprechend der des eingesetzten DNT), ca. 0,1 t/h Leicht- und Schwersieder (im Verhältnis von etwa 20 : 80) sowie ca. 3,6 t/h Wasser und bis zu etwa 1 kg/h Katalysator (v.a. Feinanteil).

Innerhalb der 2-monatigen Umsetzung nahm die mittels Röntgenpulverdiffraktometrie bestimmte Kristallitgröße des jeweils von den obigen Suspensionsproben abfiltrierten Ni-Katalysators durch Sinterung von 10 nm auf 14 nm zu.

Die DNT-Leitung wurde auch hier jeweils mit ca. 50 I VE-Wasser in Richtung Reaktor gespült.

Ein Blockieren des DNT-Ringverteilers konnte ebenfalls weder während dieses kontinuierlichen Betriebs noch zu einem anderen Zeitpunkt (z.B. bei Befüll- und Entleerschritten) detektiert werden. Bei der Routineinspektion wurde jedoch eine Polymer-, TDA-, Katalysator- und DNT-haltige Verblockung im der Zufuhrleitung abgewandten Teil des DNT-Ringverteilers festgestellt.

## Patentansprüche

1. Verfahren zur Herstellung von aromatischen Aminen durch Hydrierung von Nitroaromaten mit Wasserstoff in Gegenwart eines suspendierten Katalysators in der flüssigen Phase
- in einem Reaktor (1),
- an dessen einen, bevorzugt oberen, Ende eine Treibstrahldüse (2) angeordnet ist, über die in diesen Bereich des Reaktors (1) das aus dem Reaktorsumpf abgezogene Reaktionsgemisch über eine externe Schlaufe (3) eingedüst wird,
- mit Zuführung des Nitroaromaten an einem, bevorzugt oberen, Ende des Reaktors (1), und
- Zuführung des Wasserstoffs
**dadurch gekennzeichnet, dass**
- bei bestimmungsgemäßer Füllhöhe des Reaktors weder im Betriebszustand noch bei Abstellung ein direkter räumlicher Kontakt zwischen der Zuführung des Nitroaromaten und der flüssigen Phase, bevorzugt enthaltend das gewünschte aromatische Amin, Wasser und Katalysator, im Reaktor und/oder im Umpumpkreis erfolgen kann, und
- nach einem nicht-bestimmungsgemäßen Kontakt zwischen der Zuführung des Nitroaromaten und der Flüssigphase des Reaktors eine teilweise oder vollständige Verblockung der Zuführung des Nitroaromaten messtechnisch erfasst werden kann.

2. Verfahren nach Anspruch 1, **dadurch gekennzeichnet, dass** der Reaktor (1) vertikal aufrecht steht.

3. Verfahren nach Anspruch 1 oder 2, **dadurch gekennzeichnet, dass** das Reaktionsgemisch, welches über eine externe Schlaufe (3) eingedüst wird, anschließend in ein zentrales Einsteckrohr (4), welches in Reaktorlängsrichtung angeordnet ist, einströmt, dieses der Länge nach, bevorzugt von oben nach unten, durchströmt und in einer internen Schlaufenbewegung außerhalb des Einsteckrohrs (4) erneut nach oben strömt.

4. Verfahren nach einem der Ansprüche 1 bis 3, **dadurch gekennzeichnet, dass** ein Wärmetauscher (6) im Innenraum des Reaktors (1) vorliegt, durch den Kühlwasser strömt, und hierbei einen Teil der Reaktionswärme aufnimmt.

5. Verfahren nach einem der Ansprüche 1 bis 4, **dadurch gekennzeichnet, dass** die Zuführung des Wasserstoffs am unteren Ende des Reaktors erfolgt.

6. Verfahren nach einem der Ansprüche 1 bis 5, **dadurch gekennzeichnet, dass** die Konzentration an Wasserstoff im Reaktionsgemisch, das aus dem Reaktorsumpf in die externe Schlaufe strömt, 0,1 Vol.-%, bevorzugt 3 Vol.-%, nicht unterschreitet.

7. Verfahren nach Anspruch 4, **dadurch gekennzeichnet, dass** zusätzlich zu dem im Innenraum des Reaktors (1) angeordneten Wärmetauscher (6) ein weiterer Wärmetauscher (W) in der externen Schlaufe eingesetzt wird.

8. Verfahren nach einem der Ansprüche 1 bis 7, **dadurch gekennzeichnet, dass** die Zuführung des Nitroaromaten durch eine Zulaufleitung und/oder eine Dosiereinrichtung realisiert wird.

9. Verfahren nach Anspruch 8, **dadurch gekennzeichnet, dass** die Zuführung des Nitroaromaten über eine oder mehrere unabhängige Rohrleitungen realisiert wird, deren teilweise oder vollständige Verblockung messtechnisch erfasst werden kann.

10. Verfahren nach Anspruch 9, **dadurch gekennzeichnet, dass** der Austrittsquerschnitt der Zuführung des Nitroaromaten an den Enden der einen oder mehreren unabhängigen Rohrleitung(en) eine Verengung oder eine von der rotationssymmetrischen Form abweichende Gestalt aufweist.

11. Verfahren nach Anspruch 1 oder 2, **dadurch gekennzeichnet, dass** ein laminarer oder turbulenter Nitroaromaten-Strahl aus einer oder mehreren der Zuführungen des Nitroaromaten im Bereich des Umfangs des auf die Flüssigkeitsoberfläche treffenden Treibstrahls der Düse des äußeren Umlaufs auf die Oberfläche der flüssigen Phase aufgegeben wird.

12. Verfahren nach Anspruch 3, **dadurch gekennzeichnet, dass** ein laminarer oder turbulenter Nitroaromaten-Strahl aus einer oder mehreren der Zuführungen des Nitroaromaten im Bereich der auf die Flüssigkeitsoberfläche projizierten Querschnittsfläche des Einsteckrohres, bevorzugt möglichst nahe am Treibstrahl der Düse des äußeren Umlaufs, besonders bevorzugt direkt am Umfang des Treibstrahls der Düse des äußeren Umlaufs auf die Oberfläche der flüssigen Phase, aufgegeben wird.

13. Verfahren nach einem der Ansprüche 1 bis 12, **dadurch gekennzeichnet, dass** die Austrittsöffnungen der einen oder mehreren Zuführungen des Nitroaromaten zur Erzeugung eines radialen Impulses um bis zu 90° in Richtung der Symmetrieachse des Reaktors geneigt sind.

14. Verfahren nach einem der Ansprüche 1 bis 13, **dadurch gekennzeichnet, dass** dem in der externen Schlaufe strömenden Reaktionsgemisch zusätzlicher Wasserstoff zugeführt wird, um die Mindestkonzentration des Wasserstoffs in der externen Schlaufe sicherzustellen.

15. Verfahren nach Anspruch 6, **dadurch gekennzeichnet, dass** dem Reaktor an beliebiger Stelle Wasserstoff zugeführt wird und der Reaktordurchmesser oder die Abströmgeschwindigkeit des Reaktionsgemisches aus dem Reaktorsumpf so ausgelegt werden, dass die Mindestkonzentration des Wasserstoffs in der externen Schlaufe sichergestellt ist.

16. Verfahren nach einem der Ansprüche 1 bis 15, **dadurch gekennzeichnet, dass** die Reaktionstemperatur 80 bis 200 °C, bevorzugt 100 bis 180 °C, besonders bevorzugt 110 bis 140 °C, beträgt.

17. Verfahren nach einem der Ansprüche 1 bis 16, **dadurch gekennzeichnet, dass** der Reaktionsdruck 10 bis 50 bar absolut, bevorzugt 10 bis 30 bar absolut, besonders bevorzugt 20 bis 30 bar absolut, beträgt.

18. Verwendung einer Vorrichtung mindestens umfassend
- einen Reaktor (1)
- an dessen einen, bevorzugt oberem, Ende eine Treibstrahldüse (2) angeordnet ist, über die in diesen Bereich des Reaktors (1) das aus dem Reaktorsumpf abgezogene Reaktionsgemisch über eine externe Schlaufe (3) eingedüst werden kann,
- eine Zuführung für den Nitroaromaten an einem oberen Ende des Reaktors (1), so dass bei bestimmungsgemäßer Füllhöhe des Reaktors weder im Betriebszustand, noch bei Abstellung ein direkter räumlicher Kontakt zwischen der Zuführung des Nitroaromaten und der flüssigen Phase im Reaktor und/oder im Umpumpkreis erfolgen kann,
- eine Zuführung für den Wasserstoff und
- eine Messeinrichtung zur Bestimmung einer teilweisen oder vollständigen Verblockung der Zuführung des Nitroaromaten nach einem nicht-bestimmungsgemäßen Kontakt zwischen der Zuführung des Nitroaromaten und der Flüssigphase des Reaktors,
zur Durchführung des Verfahrens nach einem der Ansprüche 1 bis 17.

19. Verwendung nach Anspruch 18, **dadurch gekennzeichnet, dass** der Reaktor vertikal aufrecht steht.

20. Verwendung nach Anspruch 18 oder 19, **dadurch gekennzeichnet, dass** ein zentrales Einsteckrohr (4) in Reaktorlängsrichtung angeordnet ist.

21. Verwendung nach einem der Ansprüche 18 bis 20, **dadurch gekennzeichnet, dass** ein Wärmetauscher (6) im Innenraum des Reaktors (1) vorliegt, durch den Kühlwasser strömt, und hierbei einen Teil der Reaktionswärme aufnimmt.

22. Verwendung nach einem der Ansprüche 18 bis 21, **dadurch gekennzeichnet, dass** die Zuführung des Wasserstoffs am unteren Ende des Reaktors (1) erfolgt.

## Claims

1. A process for preparing aromatic amines by hydrogenation of nitroaromatics by means of hydrogen in the presence of a suspended catalyst in the liquid phase
- in a reactor (1),
- at one end of which, preferably the upper end, there is a driving jet nozzle (2) via which the reaction mixture taken off from the bottom region of the reactor is injected via an external loop (3) into this region of the reactor (1),
- with introduction of the nitroaromatic at one end, preferably the upper end, of the reactor (1) and
- introduction of hydrogen,
wherein
- at the correct fill height of the reactor, no direct physical contact between the inlet for the nitroaromatic and the liquid phase, preferably comprising the desired aromatic amine, water and catalyst, can occur in the reactor and/or in the pumped circuit either in the operating state or on shutting down and
- partial or complete blocking of the inlet for the nitroaromatic after improper contact between the inlet for the nitroaromatic and the liquid phase in the reactor can be detected by means of measuring instruments.

2. The process according to claim 1, wherein the reactor (1) is vertical.

3. The process according to claim 1 or 2, wherein the reactor mixture which is injected via an external loop (3) subsequently flows into a central plug-in tube (4) which is arranged in the longitudinal direction of the reactor, flows through the length of this, preferably from the top downward, and flows upward again in an internal loop movement outside the plug-in tube (4).

4. The process according to any of claims 1 to 3, wherein a heat exchanger (6) through which cooling water flows and takes up part of the heat of reaction is present in the interior of the reactor (1).

5. The process according to any of claims 1 to 4, wherein the hydrogen is introduced at the lower end of the reactor.

6. The process according to any of claims 1 to 5, wherein the concentration of hydrogen in the reaction mixture which flows from the bottom region of the reactor into the external loop does not go below 0.1% by volume, preferably 3% by volume.

7. The process according to claim 4, wherein a further heat exchanger (W) in addition to the heat exchanger (6) arranged in the interior of the reactor (1) is used in the external loop.

8. The process according to any of claims 1 to 7, wherein the introduction of the nitroaromatic is effected by means of a feed line and/or a metering device.

9. The process according to claim 8, wherein the introduction of the nitroaromatic is effected via one or more independent pipes in which partial or complete blocking can be detected by means of measuring instruments.

10. The process according to claim 9, wherein the exit cross section of the inlet for the nitroaromatic has a constriction or a shape deviating from a rotationally symmetric shape at the ends of the one or more independent pipe(s).

11. The process according to claim 1 or 2, wherein a laminar or turbulent nitroaromatics jet is introduced from one or more of the inlets for the nitroaromatic onto the surface of the liquid phase in the region of the perimeter of the driving jet impinging on the liquid surface from the nozzle of the external circuit.

12. The process according to claim 3, wherein a laminar or turbulent nitroaroamtics jet from one or more of the inlets for the nitroaromatic is introduced onto the surface of the liquid phase in the region of the cross-sectional area of the plug-in tube projected onto the liquid surface, preferably very close to the driving jet from the nozzle of the external circuit, particularly preferably directly at the perimeter of the driving jet of the nozzle of the external circuit.

13. The process according to any of claims 1 to 12, wherein the outlet openings of the one or more inlets for the nitroaromatic are inclined by up to 90° in the direction of the axis of symmetry of the reactor in order to generate a radial impulse.

14. The process according to any of claims 1 to 13, wherein additional hydrogen is introduced into the reaction mixture flowing in the external loop in order to ensure the minimum concentration of hydrogen in the external loop.

15. The process according to claim 6, wherein hydrogen is introduced into the reactor at any place and the reactor diameter or the outflow rate of the reaction mixture from the bottom region of the reactor is designed so that the minimum concentration of hydrogen in the external loop is ensured.

16. The process according to any of claims 1 to 15, wherein the reaction temperature is from 80 to 200°C, preferably from 100 to 180°C, particularly preferably from 110 to 140°C.

17. The process according to any of claims 1 to 16, wherein the reaction pressure is from 10 to 50 bar absolute, preferably from 10 to 30 bar absolute, particularly preferably from 20 to 30 bar absolute.

18. The use of an apparatus, which comprises at least
- a reactor (1)
- at one end of which, preferably the upper end, there is a driving jet nozzle (2) via which the reaction mixture taken off from the bottom region of the reactor can be injected via an external loop (3) into this region of the reactor (1),
- an inlet for the nitroaromatic at one upper end of the reactor (1), so that at the correct fill height of the reactor, no direct physical contact between the inlet for the nitroaromatic and the liquid phase can occur in the reactor and/or in the pumped circuit either in the operating state or on shutting down,
- an inlet for the hydrogen and
- a measuring device for determining partial or complete blocking of the inlet for the nitroaromatic after improper contact between the inlet for the nitroaromatic and the liquid phase in the reactor, for carrying out the process according to one of claims 1 to 17.

19. The use according to claim 18, wherein the reactor is vertical.

20. The use according to claim 18 or 19, wherein a central plug-in tube (4) is arranged in the longitudinal direction of the reactor.

21. The use according to any of claims 18 to 20, wherein a heat exchanger (6) through which cooling water flows and takes up part of the heat of reaction is present in the interior of the reactor (1).

22. The use according to any of claims 18 to 21, wherein the hydrogen is introduced at the lower end of the reactor (1).

## Revendications

1. Procédé de fabrication d'amines aromatiques par hydrogénation de composés nitroaromatiques avec de l'hydrogène en présence d'un catalyseur suspendu dans la phase liquide
- dans un réacteur (1)
- à une extrémité, de préférence supérieure, duquel une buse à jet d'entraînement (2) est agencée, par laquelle le mélange réactionnel soutiré du fond du réacteur est injecté dans cette zone du réacteur (1) par le biais d'une boucle externe (3),
- avec alimentation du composé nitroaromatique à une extrémité, de préférence supérieure, du réacteur (1), et
- alimentation de l'hydrogène,
**caractérisé en ce que**
- à une hauteur de remplissage normale du réacteur, un contact direct dans l'espace entre l'alimentation du composé nitroaromatique et la phase liquide, contenant de préférence l'amine aromatique souhaitée, de l'eau et le catalyseur, ne peut avoir lieu ni à l'état d'exploitation, ni à l'arrêt dans le réacteur et/ou dans le circuit de pompage, et
- après un contact non normal entre l'alimentation du composé nitroaromatique et la phase liquide du réacteur, un blocage partiel ou total de l'alimentation du composé nitroaromatique peut être déterminé par mesure.

2. Procédé selon la revendication 1, **caractérisé en ce que** le réacteur (1) est disposé à la verticale.

3. Procédé selon la revendication 1 ou 2, **caractérisé en ce que** le mélange réactionnel, qui est injecté par le biais d'une boucle externe (3), entre ensuite dans un tube d'insertion central (4), qui est agencé dans la direction longitudinale du réacteur, traverse celui-ci longitudinalement, de préférence du haut vers le bas, et s'écoule de nouveau vers le haut en un mouvement de boucle interne à l'extérieur du tube d'insertion (4).

4. Procédé selon l'une quelconque des revendications 1 à 3, **caractérisé en ce qu'**un échangeur de chaleur (6) est présent dans l'espace intérieur du réacteur (1), dans lequel de l'eau de refroidissement s'écoule et absorbe ainsi une partie de la chaleur de réaction.

5. Procédé selon l'une quelconque des revendications 1 à 4, **caractérisé en ce que** l'alimentation de l'hydrogène a lieu à l'extrémité inférieure du réacteur.

6. Procédé selon l'une quelconque des revendications 1 à 5, **caractérisé en ce que** la concentration en hydrogène dans le mélange réactionnel, qui s'écoule depuis le fond du réacteur dans la boucle externe, ne passe pas en dessous de 0,1 % en volume, de préférence 3 % en volume.

7. Procédé selon la revendication 4, **caractérisé en ce qu'**en plus de l'échangeur de chaleur (6) agencé dans l'espace intérieur du réacteur (1), un autre échangeur de chaleur (W) est utilisé dans le circuit externe.

8. Procédé selon l'une quelconque des revendications 1 à 7, **caractérisé en ce que** l'alimentation du composé nitroaromatique est réalisée par une conduite d'alimentation et/ou un dispositif de dosage.

9. Procédé selon la revendication 8, **caractérisé en ce que** l'alimentation du composé nitroaromatique est réalisée par une ou plusieurs canalisations indépendantes, dont le blocage partiel ou total peut être déterminé par mesure.

10. Procédé selon la revendication 9, **caractérisé en ce que** la section transversale de sortie de l'alimentation du composé nitroaromatique aux extrémités de la ou des canalisations indépendantes présente un rétrécissement ou une configuration différente de la forme symétrique par rotation.

11. Procédé selon la revendication 1 ou 2, **caractérisé en ce qu'**un jet de composé nitroaromatique laminaire ou turbulent issu d'une ou de plusieurs des alimentations du composé nitroaromatique est introduit sur la surface de la phase liquide dans la zone de la périphérie du jet d'entraînement atteignant la surface du liquide de la buse de la périphérie extérieure.

12. Procédé selon la revendication 3, **caractérisé en ce qu'**un jet de composé nitroaromatique laminaire ou turbulent issu d'une ou de plusieurs des alimentations du composé nitroaromatique est introduit sur la surface de la phase liquide dans la zone de la surface de section transversale projetée sur la surface du liquide du tube d'insertion, de préférence aussi près que possible du jet d'entraînement de la buse de la périphérie extérieure, de manière particulièrement préférée directement sur la périphérie du jet d'entraînement de la buse de la périphérie extérieure.

13. Procédé selon l'une quelconque des revendications 1 à 12, **caractérisé en ce que** les ouvertures de sortie de la ou des alimentations du composé nitroaromatique sont inclinées de jusqu'à 90° dans la direction de l'axe de symétrie du réacteur pour la génération d'une impulsion radiale.

14. Procédé selon l'une quelconque des revendications 1 à 13, **caractérisé en ce que** de l'hydrogène est en outre introduit dans le mélange réactionnel s'écoulant dans la boucle externe, afin d'assurer la concentration minimale de l'hydrogène dans la boucle externe.

15. Procédé selon la revendication 6, **caractérisé en ce que** de l'hydrogène est introduit dans le réacteur à un emplacement quelconque, et le diamètre du réacteur ou la vitesse d'écoulement du mélange réactionnel hors du fond du réacteur sont conçus de manière à assurer la concentration minimale de l'hydrogène dans la boucle externe.

16. Procédé selon l'une quelconque des revendications 1 à 15, **caractérisé en ce que** la température de réaction est de 80 à 200 °C, de préférence de 100 à 180 °C, de manière particulièrement préférée de 110 à 140 °C.

17. Procédé selon l'une quelconque des revendications 1 à 16, **caractérisé en ce que** la pression de réaction est de 10 à 50 bar absolu, de préférence de 10 à 30 bar absolu, de manière particulièrement préférée de 20 à 30 bar absolu.

18. Utilisation d'un dispositif comprenant au moins
- un réacteur (1),
- à une extrémité, de préférence supérieure, duquel une buse à jet d'entraînement (2) est agencée, par laquelle le mélange réactionnel soutiré du fond du réacteur est injecté dans cette zone du réacteur (1) par le biais d'une boucle externe (3),
- une alimentation pour le composé nitroaromatique à une extrémité supérieure du réacteur (1), de sorte qu'à une hauteur de remplissage normale du réacteur, un contact direct dans l'espace entre l'alimentation du composé nitroaromatique et de la phase liquide ne puisse avoir lieu ni à l'état d'exploitation, ni à l'arrêt dans le réacteur et/ou dans le circuit de pompage,
- une alimentation pour l'hydrogène, et
- un dispositif de mesure pour la détermination d'un blocage partiel ou total de l'alimentation du composé nitroaromatique après un contact non normal entre l'alimentation du composé nitroaromatique et la phase liquide du réacteur,
pour la réalisation du procédé selon l'une quelconque des revendications 1 à 17.

19. Utilisation selon la revendication 18, **caractérisée en ce que** le réacteur est disposé à la verticale.

20. Utilisation selon la revendication 18 ou 19, **caractérisée en ce qu'**un tube d'insertion central (4) est agencé dans la direction longitudinale du réacteur.

21. Utilisation selon l'une quelconque des revendications 18 à 20, **caractérisée en ce qu'**un échangeur de chaleur (6) est présent dans l'espace intérieur du réacteur (1), dans lequel de l'eau de refroidissement s'écoule et absorbe ainsi une partie de la chaleur de réaction.

22. Utilisation selon l'une quelconque des revendications 18 à 21, **caractérisée en ce que** l'alimentation de l'hydrogène a lieu à l'extrémité inférieure du réacteur (1).
